# FASCICULE DE BREVET EUROPEEN

(11) **EP 3 250 162 B1**
(45) Date de publication et mention de la délivrance du brevet: **28.11.2018**
(21) Numéro de dépôt: 16707174.5
(22) Date de dépôt: 28.01.2016
(51) Int. Cl.: A61F 5/445

(54) **DISPOSITIF DE PROTECTION DE POCHE DE STOMIE**
SCHUTZVORRICHTUNG FÜR OSTOMIEBEUTEL
OSTOMY POUCH PROTECTING DEVICE

(30) Priorité: 29.01.2015 FR 1550693
(43) Date de publication de la demande: 06.12.2017
(73) Titulaire: Barcella, Lucien, 83340 Le Cannet Des Maures (FR); Barcella, Patrick, 33990 Naujac Sur Mer (FR); Barcella, Hervé, 13410 Lambesc (FR); Barcella, David, 13002 Marseille (FR); Chodz'ko, Denys, 83170 Brignoles (FR); Somnier, Jean-Louis, 13008 Marseille (FR); Brun, Christine, 33990 Naujac Sur Mer (FR); Bertalmio, Liliane, 13410 Lambesc (FR)
(72) Inventeur: BARCELLA, Lucien, 83340 Le Cannet Des Maures (FR)
(74) Mandataire: Novagraaf Technologies
(86) Numéro de dépôt international: PCT/FR2016/050185
(87) Numéro de publication internationale: WO 2016/120569

(56) Documents cités:
- WO-A1-97/16141
- WO-A2-2013/009848
- DE-U1-202008 001 572
- DE-U1-202008 001 572
- GB-A- 2 399 502
- US-A- 2 675 002
- US-A- 4 636 206
- US-A- 5 338 315

## Description

La présente invention a pour objet un dispositif de protection de poche de stomie ou d'ostomie, et son procédé de mise en place. Le document DE 20 2008 001572 U1 représente l'état de la technique le plus proche.

Le secteur technique de l'invention est celui de la fabrication de matériel médical destiné à recueillir des fluides et/ou déchets corporels évacués par des patients.

Il est connu en effet depuis de nombreuses années, dans l'application principale de l'invention lorsque les selles d'une personne ne peuvent plus être évacuées par les voies naturelles, d'opérer chirurgicalement cette personne en pratiquant une petite ouverture, temporaire ou permanente, dans son abdomen et en y reliant l'intestin afin de permettre l'évacuation des selles par cette ouverture : celle-ci est appelée stomie, le terme d'anus artificiel étant aussi parfois utilisé. Les soignants disent également que « le côlon est abouché à la peau » (stomie vient d'ailleurs du mot grec stoma, qui signifie bouche).

On pourra se référer utilement à diverses documentations qui traitent du sujet telles que les publications de l'Institut National (Français) du Cancer dont certaines informations ci-dessous sont extraites.

Une stomie peut en effet être nécessaire dans le cadre de la chirurgie d'un cancer du côlon ou du rectum, qui est la 3eme cause de cancer chez l'homme et la 2eme chez la femme.

Après avoir enlevé la portion de côlon atteinte par la tumeur, le chirurgien procède habituellement au raccordement des deux portions de côlon non atteintes pour rétablir la continuité intestinale, mais dans certains cas, il est nécessaire de mettre le côlon au repos pour favoriser sa cicatrisation. Une colostomie temporaire est alors pratiquée. On parle aussi de colostomie de protection. Elle dure en moyenne entre trois et six mois. Une fois le côlon cicatrisé, une nouvelle intervention est programmée pour refermer la stomie et rétablir la continuité intestinale.

Une colostomie définitive peut être aussi nécessaire dans le cadre de la chirurgie d'un cancer du rectum, en particulier s'il faut enlever le sphincter anal, ce qui ne permet plus au patient d'expulser ses selles par les voies naturelles. Les selles et les gaz sont donc définitivement recueillis au niveau de l'abdomen, à travers la stomie.

Dans le cadre d'un cancer du côlon ou du rectum, c'est souvent le côlon qui est uni à la peau de l'abdomen. On parle de colostomie. La stomie peut aussi être faite au niveau de l'iléon, qui désigne la dernière partie de l'intestin grêle avant le côlon. On parle alors d'iléostomie.

La petite ouverture ainsi réalisée est de forme ronde ou ovale : la peau qui l'entoure ne contient pas de nerfs, elle n'est donc pas douloureuse lorsqu'on la touche mais elle peut saigner facilement en raison de la présence de nombreux petits vaisseaux sanguins près de la surface.

Elle s'étire légèrement pour évacuer les selles, mais contrairement à l'anus, elle ne possède pas de muscle pour contrôler l'émission des selles : celles-ci sont alors recueillies dans une poche spéciale, collée autour de la stomie et qui nécessite d'y être fixée d'une façon amovible pour être changée ou vidée, et bien sûr d'une manière étanche.

Diverses techniques ont été mis au point par plusieurs laboratoires et fabricants pour faire face à la demande des patients de plus en plus nombreux : on estime à ce jour à plus de 800.000 personnes concernées en France, 12 Millions en Europe et plus de 60 Millions dans le Monde ! Pour améliorer l'efficacité, la fiabilité et la mise en oeuvre de ces poches de stomie, de nombreuses demandes de brevets ont été déposées et publiées depuis plus de 40 ans et on pourra s'y reporter utilement sans qu'il soit besoin de décrire ces techniques dans la présente description.

On peut citer par exemple dans ce domaine le brevet FR2387643 publié le 17/11/1978 au nom de la sté US Squibb & Sons sur un « dispositif d'ostomie », ou le brevet EP0832626 publié le 01/04/1998 au nom de la sté US Hollister Incorporation (qui a déposé plus de 110 demandes de brevets sur le sujet) sur un « dispositif d'ostomie et sa couronne de fixation adhésive associé », ou encore le brevet EP1913908 publié 23/04/2008 au nom de la sté Coloplast (qui a déposé plus de 180 demandes de brevets sur le sujet depuis 1974) sur un « dispositif pour stomie ».

Malgré cependant ces nombreuses techniques et demandes de brevets, de telles poches subissent la pression des vêtements et peuvent déborder, et leur port est assez inconfortable et inesthétique, leur volume les rendant trop visibles, avec des risques d'arrachage du fait qu'elles représentent une excroissance importante tenue par leur seul orifice, et donc de débordement : ces inconvénients empêchent les patients de vivre à l'extérieur de chez eux, bloqués également psychologiquement par l'appréhension, l'inconfort et l'image qu'ils ont d'eux même.

Aussi il a été étudié des dispositifs de soutien, de support et/ou de protection de ces poches, dont certains développés récemment, pour pallier à ces inconvénients, tels que :
- des ceintures ou des sous-vêtements aptes à soutenir:
   × soit directement ces poches tels que décrits par exemple dans la demande de brevet US 2012/0283679 de Mary Berish publiée le 08/11/2012 ou la demande WO 2014/082147 de la sté brésilienne LP da Mata publiée le 05/06/2014
   × soit indirectement tel que décrit dans le brevet US 5651777 de Wanda Walters publié le 29/07/1997 et qui enseigne un porte-poche fixé à une ceinture et ouvert latéralement pour y glisser la poche.
- des coques de protection des poches telles que décrites par exemple :
   × dans la demande internationale PCT2013/009848 dans laquelle il est enseigné une coquille rigide en forme de cuvette solidaire d'un anneau de base fermé, et laissant entre eux une ouverture dimensionnée pour permettre d'y glisser une poche de collecte en l'introduisant par le dessous, quand le patient est en position debout, l'anneau de base annulaire venant s'appuyer sur la couronne de fixation de la poche sur une pièce support collée à la peau,
   × ou encore dans le modèle d'utilité allemand DE202008001572 U dans lequel il est enseigné une grille en forme de dôme qui vient se fixer sur une pièce support de la poche constituée par un demi-cylindre en plastique, ladite grille protégeant alors la poche contre des pressions à travers les vêtements.

Cependant ces dispositifs ne sont pas satisfaisants car pour les ceintures ou sous-vêtements :
- soit ils compriment trop les poches, ce qui d'une part empêche la bonne évacuation des selles dans celles-ci (créant un inconfort accru pour le patient avec en plus un risque de débordement si la poche se détache à cause de la pression) et d'autre part, comme indiqué en introduction, peut faire saigner la peau qui entoure la stomie,
- soit ils ne soutiennent pas assez les poches, dont les problèmes évoquées précédemment restent alors entiers.

Et pour les coques de protection :
- soit entre autres il n'est pas possible de les enlever quand la poche est pleine, ce qui est très contraignant pour le patient,
- soit elles ne sont pas suffisamment guidées par rapport à la poche, ne sont pas bien maintenues sur celle-ci et donc peuvent se déplacer latéralement, ou/et n'empêchent pas les fuites de la poche au niveau de sa fixation et/ou nécessitent une pièce intermédiaire additionnelle soit pour la fixation, soit pour absorber justement les fuites éventuelles.

Le problème posé et les objectifs de l'invention sont donc de permettre, à la fois :
- un bon soutien de la poche de stomie au plus près du corps du patient pour, d'une part empêcher toute fuite de la poche au niveau de sa fixation et d'autre part effacer le plus possible son excroissance et rendre une esthétique acceptable pour celui-ci, et
- d'assurer un bon maintien de la poche et de pouvoir être enlevé facilement même quand cette poche est pleine,
- ainsi que de permettre une bonne évacuation des selles dans la poche avec un minimum de risque de fuite et/ou de saignement ; et
- tout cela sans perturber la mise en place et le maintien des poches sur et autour des orifices de stomie, quel que soit le type de poches connues utilisées,
- avec pour objectif final de permettre aux patients de pouvoir sortir de chez eux sans crainte et avec un confort optimum, et ainsi de vivre quasi normalement.

Une solution au problème posé est un dispositif de protection de poche de stomie, destiné à être fixé sur une pièce support comprenant deux couronnes reliées par leur diamètre intérieur formant une gorge tournée vers l'extérieur, et dont l'une est collée autour de l'orifice d'une stomie et sur la peau d'un patient ; lequel dispositif comporte une coquille rigide, creuse et apte à venir recouvrir la dite poche, et une couronne ou anneau de maintien solidaire du bord de ladite coquille, et dont la partie centrale évidée est, d'une part, de dimension plus petite que celle de la forme extérieure de la poche et, d'autre part, apte à permettre le passage de l'orifice d'entrée de la poche une fois celle-ci connectée à la pièce support, et tel que suivant l'invention :
- la couronne de maintien est ouverte sur une partie de sa périphérie et est apte à permettre ainsi, lors de la mise en place du dispositif sur la poche, de faire glisser la dite couronne en arrière de la poche et par le dessus, quand le patient est en position debout, et autour de la gorge de la pièce support assurant la connexion de l'orifice d'entrée de la poche sur cette pièce support et d'y accrocher le dispositif,
- la couronne de maintien comporte une languette amovible qui ferme l'ouverture de la partie ouverte de la périphérie de la couronne, une fois celle-ci mise en place autour de la gorge de la pièce support, empêchant toute possibilité de déplacer et à plus fortes raisons d'enlever le dispositif de protection par glissement latéral ou vertical, ladite couronne et sa languette amovible en position fermée étant aptes à entourer complètement ladite gorge.

De par cette conception décrite plus en détail ci-après, la mise en place et l'enlèvement d'un tel dispositif est facile, rapide et simple, sans avoir à ôter en même temps la poche et sans en perturber le fonctionnement, et, celle-ci restant ainsi libre au niveau de la stomie et sans compression puisque protégée par la coquille rigide, l'évacuation des selles n'est pas perturbée.

De plus la cavité formée et délimitée par la coquille creuse rigide est de préférence de faible épaisseur grâce à une courbure assez plate de la forme de la coquille, ce qui donne au dispositif un encombrement d'excroissance minimum par rapport à la peau du patient, tout en laissant un volume intérieur suffisant pour contenir la poche de stomie ou tout au moins sa partie supérieure, même pleine, sans compression.

Dans un mode préférentiel de réalisation, le dispositif comporte une ceinture apte à appuyer sur la coquille et à plaquer la couronne de maintien contre la peau, ce qui permet, d'une part d'empêcher toute fuite de la poche au niveau de sa fixation sur la pièce support collée à la peau et d'autre part d'effacer le plus possible l'excroissance de la poche sans l'écraser grâce à la rigidité de la coquille qui la protège, et de donner ainsi une esthétique acceptable pour le patient.

Une autre solution pour atteindre les objectifs de l'invention est un procédé de mise en place spécifique qui permet de répondre au problème posé d'une manière plus certaine que les caractéristiques propres du dispositif de protection de poche de stomie :
- d'une manière connue, on fixe une poche de stomie sur une pièce support comprenant deux couronnes reliées par leur diamètre intérieur formant une gorge tournée vers l'extérieur et dont on colle une de ces couronnes autour de l'orifice d'une stomie sur la peau d'un patient,
- on vient recouvrir ladite poche par une coquille rigide et creuse solidaire d'une couronne ou anneau de maintien et dont la partie centrale évidée est d'une part de dimension plus petite que celle de la forme extérieure de la poche et d'autre part apte à permettre le passage de l'orifice d'entrée de cette poche quand on a connecté celle-ci à la pièce support, et suivant l'invention :
- on fait glisser la couronne de maintien qui est ouverte sur une partie de sa périphérie, en arrière de la poche et de haut en bas quand le patient est en position debout, la partie supérieure au moins de la poche se logeant ainsi dans la partie centrale évidée de la coquille,
- on arrête le glissement de la couronne de maintien quand celle-ci est disposée autour de la gorge de la pièce support assurant la connexion de l'orifice d'entrée de la poche sur cette pièce support et on y accroche le dispositif,
- on ferme l'ouverture de la partie ouverte de la périphérie de la couronne de maintien par une languette amovible une fois cette couronne mise en place autour de la gorge, la dite couronne et sa languette amovible en position ainsi fermée entourant alors complètement la gorge et empêchant toute possibilité de déplacement de la coquille de protection par glissement latéral ou vertical,
- on applique une ceinture sur la dite coquille et qui entoure le corps du patient, et en appuyant ainsi par serrage de la ceinture sur cette coquille, on plaque la couronne de maintien contre la peau en prenant en « sandwich » la couronne de la pièce support qui est collée à la peau, empêchant tout risque de fuite.

De plus le dispositif de protection suivant l'invention peut comporter des plaques latérales débordant de la couronne de maintien au-delà de la coquille, formant ainsi des sortes d'oreilles et permettant de comprimer, en même temps que le soutien sans compression et la protection de la poche, toutes excroissances d'hernies, souvent provoquées par l'opération chirurgicale et/ou les traitements associés, et qui gênent également les patients.

Un mode de réalisation particulier est tel que la coquille et la couronne de maintien sont en deux parties assemblables solidairement l'une à l'autre, l'une des parties de la couronne de maintien faisant office de languette amovible et les deux parties une fois assemblées entourent et couvrent complètement la poche en formant une coquille entièrement fermée, ce qui permet d'y enfermer et isoler complètement une petite poche et de permettre au patient de pratiquer tout sport en toute sécurité, dont en particulier la baignade.

Le résultat est un nouveau dispositif de protection et de support de poche de stomie qui répond à l'ensemble des aspects du problème posé ci-dessus et dont les avantages et les caractéristiques évoqués ci-dessus et décrits ci-après en prouvent l'intérêt. En effet un tel dispositif permet à toute personne « stomisée », et portant une poche de stomie protégée par ce dispositif, de se vêtir normalement, d'aller et venir sans contrainte comme bon lui semble, lui apportant une décontraction vis-à-vis d'autrui, et lui permettant ainsi de vivre chaque instant sans appréhension du pire comme les systèmes proposés à ce jour.

Un prototype a été réalisé et testé à titre personnel et confidentiel par l'inventeur lui-même, ce qui a permis de vérifier le bien être apporté et de contrôler le bon fonctionnement du dispositif et de constater ni complication médicale (tel qu'aucune torsion, aucun effet baïonnette), ni effet contraire au bon fonctionnement de la stomie ; de plus il s'avère que lorsqu'on porte le dispositif suivant la présente invention, celui-ci sert de régulateur des déjections fécales comme le fait un anus ; il a également été vérifié que le fait de le placer sur le pourtour du support de stomie, et de le plaquer sur la peau avec en plus une ceinture d'appui, empêche le décollement de ce support et le décrochage de la poche, et évite ainsi toute fuite autour des stomies, ce qu'aucun dispositif connu à ce jour ne permet d'assurer.

Il est à noter également que si la poche de stomie est pleine, le fait d'avoir une languette amovible qui s'ouvre vers le bas permet d'enlever le dispositif rapidement et sans problème et d'accéder ainsi à la poche, ce qui peut être nécessaire en cas d'intervention rapide (comme lors de diarrhées et de problèmes gastriques qui provoquent un gonflement rapide de la poche). Le dispositif suivant l'invention permet aussi au patient de porter de petites poches très confortables et esthétiques, et du fait des caractéristiques de la présente invention, celles-ci n'ont pas besoin d'être vidangées car leur remplacement peut se faire dans n'importe quel lieu un peu isolé.

Le dispositif de protection de poche suivant l'invention apporte ainsi une amélioration certaine pour le confort et le bien être des personnes ou patients « stomisés » : ceux-ci peuvent porter un tel dispositif suivant leurs besoins personnels ressentis, même avec des poches de stomie portées continuellement avec leur différentes fonctions d'utilisation ; le dispositif suivant l'invention assure une sécurité contre les fuites éventuelles et/ou le décollage du support collé à la peau sans pour autant blesser et faire saigner et/ou perturber la stomie : son utilisation est ainsi principalement conseillée et utile pour les déplacements, les sorties, les vacances, les petits travaux, le sport dont en particulier la baignade, ... que peut ainsi faire le patient équipé d'un dispositif suivant l'invention.

De plus, un tel dispositif peut être utilisé dans toute autre situation où un orifice a été pratiqué chirurgicalement sur un patient pour créer et remplacer un orifice naturel tel que dans des applications urinaires, dites urostomie.

La description et les figures ci-jointes en donnent deux exemples de réalisation mais d'autres modes de réalisation sont possibles dans le cadre de la portée de la présente invention, en notant que le dispositif suivant l'invention peut être réalisé pour tout type et volume de poche de stomie existante ou à venir, et tout diamètre de stomie tel qu'à ce jour 50 et 60mm.

La figure 1 est une vue de face d'un exemple de dispositif suivant l'invention représenté dans une position verticale correspondant à sa mise en place sur une poche de stomie et posé en contact contre la peau d'un patient considéré debout, déterminant ainsi son positionnement dans l'espace à partir duquel sont référencées les vues des figures 2 à 4.

La figure 2 est une vue en perspective de trois quart de la face arrière, considérée par rapport à la figure 1 et venant en contact contre la peau d'un patient, du même exemple de dispositif suivant l'invention mais sans poche ni ceinture.

La figure 3 est une vue en perspective, prise de trois quart de face et par en dessous du dispositif suivant l'invention, considéré par rapport à la figure 2, et en position d'ouverture pour être mis en place sur une poche de stomie.

La figure 4 est une vue de profil en perspective, prise par en dessous d'un dispositif suivant l'invention, considéré par rapport à la figures 3, et en position de fermeture.

La figure 5 est une coupe transversale horizontale suivant V,V' de la figure 1 schématisant un dispositif suivant l'invention mis en place sur une poche de stomie et posé en contact contre la peau d'un patient.

La figure 6 est une vue de face d'un autre exemple de dispositif suivant l'invention représenté dans une position verticale correspondant à sa mise en place sur une poche de stomie et posé en contact contre la peau d'un patient considéré debout.

Le dispositif de protection de poche 3 de stomie comporte suivant l'invention une coquille 1 rigide, creuse et apte à venir recouvrir la dite poche 3, et une couronne ou anneau de maintien 10 solidaire du bord de ladite coquille 1, et dont la partie centrale évidée 22 est apte à permettre le passage de l'orifice d'entrée 12 de la poche 3 une fois celle-ci connectée à l'orifice de stomie 8 de passage des selles à travers la peau 13 d'un patient.

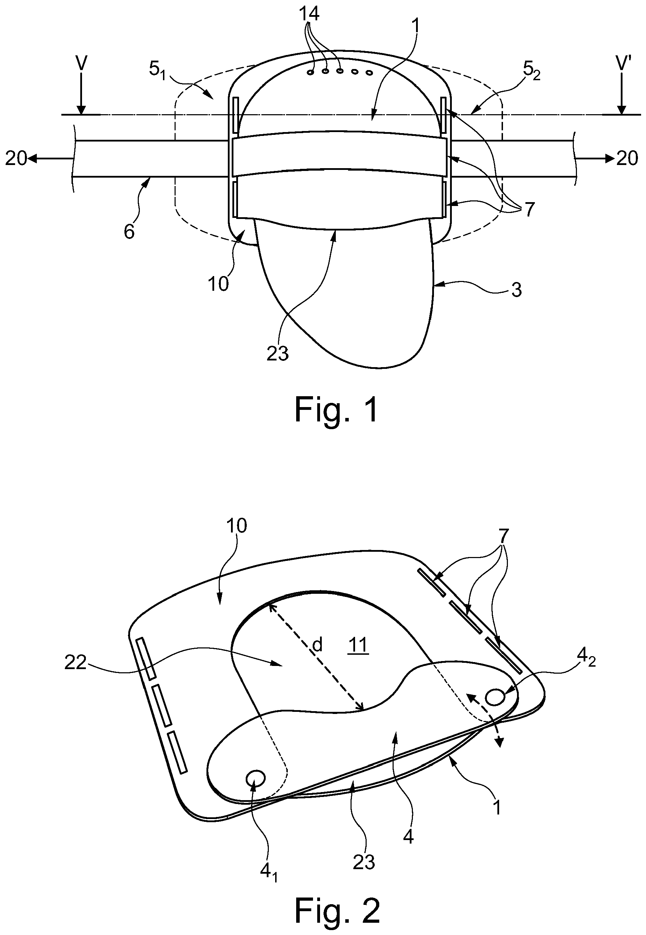

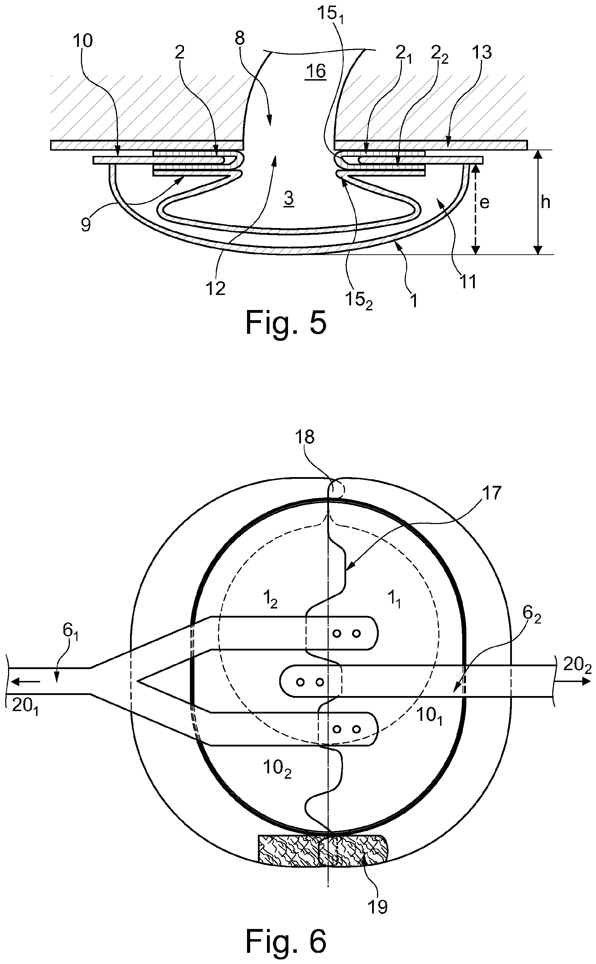

La cavité 11 formée et délimitée par la coquille creuse rigide 1 crée un logement où la poche 3 est insérée librement, soit au moins pour sa partie supérieure (considérée en position verticale sur un patient debout comme représentée sur la figure 1) soit totalement comme c'est obligatoirement le cas suivant la figure 6 ; la cavité 11 est de préférence de faible épaisseur « e » grâce à une courbure assez plate de la forme de la coquille 1, ce qui donne au dispositif un encombrement ou hauteur « h » d'excroissance minimum par rapport à la peau 13 du patient, tout en laissant dans l'exemple de réalisation des figures 1 à 4, un volume intérieur suffisant pour contenir la partie haute de la poche 3 de stomie, même pleine, sans compression.

Selon l'invention tel que représentée sur les figures 1 à 4, la couronne ou anneau de maintien 10 est ouverte sur une partie 21 de sa périphérie pour permettre, lors de la mise en place du dispositif sur la poche 3 préalablement fixée sur la stomie 8 du patient, de faire glisser parallèlement à la peau de celui-ci et par le dessus, du haut vers le bas quand le patient est debout, la dite couronne 10 autour de la connexion de l'orifice d'entrée 12 de la poche 3 sur la pièce support 2 de la poche 3, comme représenté sur la figure 5, et d'y accrocher le dispositif : cet orifice d'entrée 12 de la poche et relié directement à l'orifice 8 de passage des selles, ou stomie, à laquelle a été relié chirurgicalement l'intestin 16, à travers la peau 13 du patient ; la connexion étanche entre ces deux orifices 12,8 est assuré d'une manière connue par :
- d'un côté la pièce support 2 entourant la stomie 8, en forme de « diabolo » composé de deux couronnes 2₁, 2₂ reliées par leur diamètre intérieur et formant une gorge ou col 15₁ entre elles, et dont une première couronne 2₁ est collée à la peau, et
- de l'autre côté une couronne de fixation 9 faisant partie intégrante de la poche 3 et adhérant d'une manière connue, réversible et étanche à la deuxième couronne 2₂ de la pièce support 2.

Le dispositif suivant l'invention est glissé autour de cette gorge 15₁ formant une sorte de col périphérique entre les deux couronnes 2₁, 2₂ de cette pièce support 2, ce qui permet de ne pas solliciter la poche 3 et d'assurer une meilleure tenue de celle-ci sans risque d'arrachement.

Le bord intérieur de la couronne 9 de fixation de la poche 3 est solidaire (tel que par moulage si l'ensemble forme une seule pièce) de l'orifice 12 de la poche 3 : cette liaison forme ainsi également une gorge 15₂ extérieure formant un col périphérique entre la couronne 9 et la poche 3 ; cette gorge 15₂ comme celle 15₁ de la pièce support 2permettraitau dispositif suivant l'invention d'y être aussi glissé et guidé, comme cela est enseigné dans des dispositifs antérieurs, mais ce mode de mise en place n'est pas satisfaisant car le dispositif de protection sollicite et tire alors sur la poche 3 et peut provoquer ainsi des fuites.

Le diamètre intérieur « d » de la partie centrale évidée 22 de la couronne ou anneau de maintien 10 du dispositif de protection est d'un diamètre légèrement plus important que le diamètre extérieur de la gorge 15₁ pour venir entourer celle-ci au plus près et permet ainsi d'exercer une pression annulaire sur le pourtour de fixation de la poche 3 et au plus près de l'orifice de stomie 8 : cette disposition assure d'une part une sécurité contre le décollement de la pièce support 2 collée à la peau et d'autre part une régulation des déjections des selles comme un anus (cet effet a été constaté comme indiqué précédemment et expliqué par les docteurs qui ont suivi l'expérimentation de M. Barcella).

La conception du dispositif suivant l'invention permet d'éviter tout angle et tout espace mort, tout en offrant une accessibilité maximum pour le nettoyage.

De plus la couronne ou anneau de maintien 10 comporte une languette amovible 4, représentée ici sur les figures 2 à 4 pivotante autour d'une de ses extrémités 4₁ articulée sur un coté de l'ouverture extérieure 21 de la couronne 10, et clipsable à son autre extrémité 4₂ sur l'autre côté de l'ouverture 10₁.

Cette languette 4 est ainsi apte à fermer cette ouverture 21 d'une partie de la périphérie de la couronne 10, une fois celle-ci mise en place autour de la gorge 15₁ de la pièce support 2 correspondant à l'orifice d'entrée 12 de la poche 3, et telle que représentée sur la figure 1.

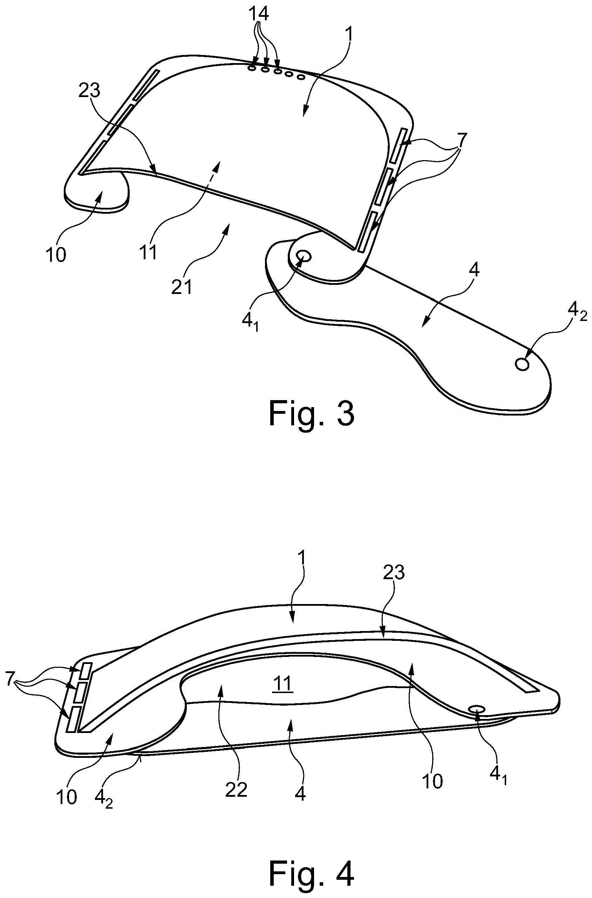

La dite couronne 10 et sa languette amovible 4 en position fermée sont ainsi aptes à entourer complètement la gorge 15₁ formée par les deux couronnes 2₁ et 2₂ reliées par leur diamètre intérieur et constituant la pièce support 2 collée à la peau par l'une des dites couronnes qui la constituent ; ceci rend le dispositif de protection solidaire de la pièce support 2 et empêche toute possibilité de déplacer le dispositif de protection par glissement latéral ou vertical ; les figures 2 et 4 représentent aussi le dispositif avec la languette 4 en position de fermeture, mais sans la poche 3 ni la ceinture 6 figurant sur la figure 1.

Cette languette 4 assure la tenue à la fois de la poche 3 et de l'appareillage de la stomie sans torsion ni effort baïonnette, même si la poche est plus longue que la coquille 1 et dépasse vers le bas (comme représenté sur la figure 1 quand le patient est debout) en passant entre la coquille 1 et cette languette 4 : pour cela, une partie 23 du bord périphérique de la coquille 1 est ouvert à l'aplomb de l'ouverture 21 de la couronne ou anneau de maintien 10 pour permettre le passage de la poche 3, une fois le dispositif positionné sur celle-ci et la languette 4 refermée ; et le reste du bord périphérique de la coquille 1 est bien sûr solidaire de la couronne de maintien 10.

Le dispositif suivant l'invention comporte au moins une ceinture 6, souple, apte à entourer le corps du patient, à appuyer, quand on la serre, sur la coquille 1 rigide, et donc sans écraser la poche 3, et à plaquer la couronne de maintien 10 contre la peau 13. Pour la maintenir en place sur la coquille 1 le dispositif peut comporter au moins un jeu de deux passants 7 situés chacun sur un coté de la couronne 10 de part et d'autre de la coquille 1 et dans un plan horizontal quand celle-ci est considérée mise en place sur un patient debout.

Ainsi on peut considérer la mise en place spécifique du dispositif de protection tel que décrit ci-dessus suivant les opérations suivantes, une fois la poche 3 de stomie fixée sur sa pièce support 2 :
- on vient recouvrir ladite poche 3 par une coquille 1 rigide et creuse solidaire d'une couronne ou anneau 10 de maintien et dont la partie centrale évidée 22 est d'une part de dimension plus petite que celle de la forme extérieure de la poche 3 et d'autre part apte à permettre le passage de l'orifice d'entrée 12 de la poche 3 quand on a connecté celle-ci à la pièce support 2,
- on fait glisser la couronne de maintien 10 qui est ouverte sur une partie 21 de sa périphérie, en arrière de la poche 3 et de haut en bas quand le patient est en position debout, la partie supérieure au moins de la poche se logeant ainsi dans la partie centrale évidée 22 de la coquille 1,

- on arrête le glissement de la couronne de maintien 10 quand celle-ci est disposée autour de la gorge 15₁ de la pièce support 2 assurant la connexion de l'orifice d'entrée 12 de la poche 3 sur cette pièce support 2 et on y accroche le dispositif,
- on ferme l'ouverture 21 de la partie ouverte de la périphérie de la couronne de maintien 10 par une languette amovible 4 une fois cette couronne 10 mise en place autour de la gorge 15₁, la dite couronne 10 et sa languette amovible 4 en position ainsi fermée entourant alors complètement la gorge 15₁ et empêchant toute possibilité de déplacer la coquille de protection par glissement latéral ou vertical,
- on applique une ceinture 6 sur la dite coquille 1 et qui entoure le corps du patient, et en appuyant ainsi par serrage de la ceinture 6 sur cette coquille, on plaque la couronne 10 de maintien contre la peau en prenant en « sandwich » la couronne 2₁ de la pièce support 2 qui est collée à la peau, empêchant tout risque de fuite.

De préférence le dispositif comporte au moins deux jeux, et même trois comme représentés sur les figures 1 à 4, de deux passants 7 permettant un réglage de position de la ceinture 6 par rapport à la coquille 1 suivant le type de pantalon porté par le patient et la position de la stomie.

En effet les passants 7 du haut sont à utiliser quand le patient porte un pantalon taille basse, ceux du bas pour un pantalon taille haute, ceux du milieu pour des vêtements à taille élastique, short ou survêtement, afin d'avoir la couronne 10 toujours maintenue bien à plat contre la peau 13.

Le dispositif peut comporter aussi des plaques d'appui latérales 5 débordant de la couronne de maintien 10 au-delà et de part et d'autre de la coquille 1, sauf bien sûr du côté de l'ouverture 10₁ de la couronne : ces plaques peuvent être des extensions de la couronne 10 dont elles forment alors des sortes d'oreilles et comme représenté en pointillé sur la figure 1 ; ces plaques en forme d'oreilles 5 permettent ainsi de comprimer toutes excroissances d'hernies pouvant exister autour de la stomie.

La coquille 1 comporte également au moins un trou d'évent 14, et de préférence plusieurs tels que cinq représentés sur les figures 1 et 3, percé dans sa partie considérée haute quand elle est mise en place sur un patient debout tel que représenté sur la figure 1 : ces trous d'évent 14 permettent l'évacuation, hors de la coquille 1, des gaz éventuels pouvant s'échapper des poches 3, qui comportent du reste un filtre pour cela ; ces trous d'évent 14 assurent également une certaine insonorisation.

Suivant un autre mode de réalisation représenté sur la figure 6 pour une protection complète de la poche 3 lors de baignade en particulier, la coquille 1 et la couronne de maintien 10 sont en deux parties assemblables solidairement l'une à l'autre, et de préférence articulées l'une à l'autre autour d'un axe commun 18 situé en partie haute: l'une de ces parties faisant office de la languette amovible des figures 1 à 4 décrites précédemment et l'autre partie faisant office de la couronne ouverte du mode de réalisation de ces dites figures.

Ces deux parties une fois assemblées solidairement entourent et couvrent complètement la poche 3 qui est alors de petite dimension bien entendu pour être entièrement contenue dans le logement 11 formé par la cavité de la coquille creuse 1 rigide, qui doit être le plus étanche possible et ne comporte du reste pas d'évents.

Pour assurer une bonne continuité de la surface de la coquille 1 une fois fermée, les bords des deux parties de la coquille 1 formant une ligne de jonction 17 d'assemblage de celle-ci sont de forme complémentaires et s'emboitent l'un par rapport à l'autre, tels que de forme ondulée suivant une sinusoïde comme représenté sur la figure 6. La fermeture entre les deux parties de la coquille doit être en effet la plus étanche possible et est par ailleurs maintenue :
- d'une part par un système de maintien 19, tel que par clip ou bandes auto-accrochantes, disposé en partie basse à l'opposé de l'articulation 18, et
- d'autre part par la ceinture 6 qui est interrompue au niveau de la coquille 1 et dont les deux extrémités 6₁, 6₂ formées par cette interruption sont chacune fixée respectivement sur la demi-coquille opposée au sens de traction 20 de l'extrémité considérée, l'une d'entre elles 6₁ étant doublée pour être fixée de part et d'autre de l'autre 6₂ et permettre que les tractions 20₁, 20₂ sur chaque extrémité 6₁, 6₂ de la ceinture soient alignées et ne fassent pas tourner le dispositif.

## Revendications

1. Dispositif de protection de poche (3) de stomie, destinée à être fixée sur une pièce support (2) comprenant deux couronnes reliées (2₁, 2₂) par leur diamètre intérieur formant une gorge (15₁) tournée vers l'extérieur dont l'une est apte à être collée autour de l'orifice (8) d'une stomie et sur la peau d'un patient, lequel dispositif comportant une coquille (1) rigide, creuse et apte à venir recouvrir la dite poche (3), et une couronne ou anneau de maintien (10) solidaire du bord de ladite coquille (1), et dont la partie centrale évidée (22) est, d'une part, de dimension plus petite que celle de la forme extérieure de la poche (3) et, d'autre part, apte à permettre le passage de l'orifice d'entrée (12) de la poche (3) une fois celle-ci connectée à la pièce support (2) la couronne de maintien (10) étant ouverte sur une partie (21) de sa périphérie et étant apte à permettre ainsi, lors de la mise en place du dispositif sur la poche (3), de faire glisser ladite couronne (10) en arrière de la poche (3) et par le dessus, quand le patient est en position debout, et dans la gorge (15₁) de la pièce support (2) assurant la connexion de l'orifice d'entrée (12) de la poche (3) sur cette pièce support (2) et d'y accrocher le dispositif, **caractérisé en ce que** :
- la couronne de maintien (10) comporte une languette amovible (4) qui ferme l'ouverture (21) de la partie ouverte de la périphérie de la couronne (10) une fois celle-ci mise en place autour de la gorge (15₁), empêchant toute possibilité de déplacer le dispositif de protection par glissement latéral ou vertical, la dite couronne (10) et sa languette amovible (4) en position fermée étant aptes à entourer complètement la gorge (15₁) .

2. Dispositif de protection suivant la revendication 1 **caractérisé en ce qu'**il comporte au moins une ceinture (6) apte à appuyer sur la coquille (1) et à plaquer la couronne de maintien (10) contre la peau (13).

3. Dispositif suivant la revendication 2 **caractérisé en ce qu'**il comporte au moins deux jeux de deux passants (7) permettant un réglage de position de la ceinture (6) par rapport à la coquille (1).

4. Dispositif suivant l'une quelconque des revendications 1 à 3 **caractérisé en ce qu'**il comporte des plaques latérales (5) débordant de la couronne de maintien (10) au-delà de la coquille (1).

5. Dispositif de protection suivant l'une quelconque des revendications 1 à 4 **caractérisé en ce que** la coquille (1) comporte au moins un trou d'évent (14) percé dans sa partie considérée haute quand elle est mise en place sur un patient debout.

6. Dispositif de protection suivant l'une quelconque des revendications 1 à 5 **caractérisé en ce qu'**une partie (23) du bord périphérique de la coquille (1) est ouvert à l'aplomb de l'ouverture (21) de la couronne de maintien (10), et est apte à permettre le passage de la poche (3), une fois le dispositif positionnée sur celle-ci et la languette (4) refermée.

7. Dispositif de protection suivant l'une quelconque des revendications 1 à 6 **caractérisé en ce que** la coquille (1) et la couronne de maintien (10) sont en deux parties assemblables solidairement l'une à l'autre, l'une des parties de la couronne de maintien faisant office de languette amovible (4).

8. Dispositif de protection suivant la revendication 7 **caractérisé en ce que** les deux parties une fois assemblées entourent et couvrent complètement la poche (3).

9. Dispositif suivant la revendication 7 ou 8 **caractérisé en ce que** les bords des deux parties de la coquille (1) formant une ligne de jonction (17) d'assemblage de celle-ci sont de forme complémentaires et s'emboitent l'un par rapport à l'autre.

10. Procédé de mise en place d'un dispositif de protection de poche de stomie (3) que l'on fixe sur une pièce support (2) comprenant deux couronnes (2₁, 2₂) reliées par leur diamètre intérieur formant une gorge (15₁) tournée vers l'extérieur et dont on colle une (2₁) de ces couronnes autour de l'orifice (8) d'une stomie sur la peau d'un patient et on vient recouvrir ladite poche (3) par une coquille (1) rigide et creuse solidaire d'une couronne ou anneau (10) de maintien et dont la partie centrale évidée (22) est d'une part de dimension plus petite que celle de la forme extérieure de la poche (3) et d'autre part apte à permettre le passage de l'orifice d'entrée (12) de la poche (3) quand on a connecté celle-ci à la pièce support (2) **caractérisé en ce que** :
- on fait glisser la couronne de maintien (10) qui est ouverte sur une partie (21) de sa périphérie, en arrière de la poche (3) et de haut en bas quand le patient est en position debout, la partie supérieure au moins de la poche se logeant ainsi dans la partie centrale évidée (22) de la coquille (1),
- on arrête le glissement de la couronne de maintien (10) quand celle-ci est disposée autour de la gorge (15₁) de la pièce support (2) assurant la connexion de l'orifice d'entrée (12) de la poche (3) sur cette pièce support (2) et on y accroche le dispositif,
- on ferme l'ouverture (21) de la partie ouverte de la périphérie de la couronne de maintien (10) par une languette amovible (4) une fois cette couronne (10) mise en place autour de la gorge (15₁), la dite couronne (10) et sa languette amovible (4) en position ainsi fermée entourant alors complètement la gorge (15₁) et empêchant toute possibilité de déplacer la coquille de protection par glissement latéral ou vertical,
- on applique une ceinture (6) sur la dite coquille (1) et qui entoure le corps du patient, et en appuyant ainsi par serrage de la ceinture (6) sur cette coquille, on plaque la couronne (10) de maintien contre la peau en prenant en « sandwich » la couronne (2₁) de la pièce support (2) qui est collée à la peau, empêchant tout risque de fuite.

## Patentansprüche

1. Vorrichtung zum Schutz eines Stomabeutels (3), der dazu vorgesehen ist, an einem Trägerteil (2) befestigt zu werden, das zwei über ihren Innendurchmesser verbundene Kränze (2₁, 2₂) umfasst, die eine zur Außenseite gerichtete Kehle (15₁) bilden, von denen einer in der Lage ist, um die Öffnung (8) eines Stomas herum und auf die Haut eines Patienten geklebt zu werden, wobei die Vorrichtung eine starre Schale (1) umfasst, die hohl und in der Lage ist, den Beutel (3) abzudecken, und einen Haltekranz oder -ring (10), der fest mit dem Rand der Schale (1) verbunden ist und dessen mittlerer ausgesparter Teil (22) einerseits von kleinerer Abmessung ist als jener der Außenform des Beutels (3), und andererseits in der Lage ist, den Durchtritt der Einlassöffnung (12) des Beutels (3) zu ermöglichen, sobald dieser mit dem Trägerteil (2) verbunden ist, wobei der Haltekranz (10) an einem Teil (21) seines Umfangs offen ist und in der Lage ist, so beim Anbringen der Vorrichtung am Beutel (3) zu ermöglichen, den Kranz (10) hinter dem Beutel (3) und von oben, wenn sich der Patient in aufrechter Position befindet, und in die Kehle (15₁) des Trägerteils (2) zu schieben, wobei sie die Verbindung der Einlassöffnung (12) des Beutels (3) an diesem Trägerteil (2) sicherstellt, und die Vorrichtung dort einzuhängen, **dadurch gekennzeichnet, dass**:
- der Haltekranz (10) eine entfernbare Lasche (4) umfasst, die die Öffnung (21) des offenen Teils des Umfangs des Kranzes (10) schließt, sobald dieser um die Kehle (15₁) herum angebracht ist, wobei sie jede Möglichkeit verhindert, die Schutzvorrichtung durch seitliches oder vertikales Verschieben zu bewegen, wobei der Kranz (10) und seine entfernbare Lasche (4) in geschlossener Position in der Lage sind, die Kehle (15₁) vollständig zu umschließen.

2. Schutzvorrichtung nach Anspruch 1, **dadurch gekennzeichnet, dass** sie mindestens einen Gurt (6) umfasst, der in der Lage ist, auf die Schale (1) zu drücken und den Haltekranz (10) gegen die Haut (13) zu pressen.

3. Vorrichtung nach Anspruch 2, **dadurch gekennzeichnet, dass** sie mindestens zwei Sätze von zwei Durchführungen (7) umfasst, die eine Positionsregelung des Gurtes (6) in Bezug auf die Schale (1) ermöglichen.

4. Vorrichtung nach einem der Ansprüche 1 bis 3, **dadurch gekennzeichnet, dass** sie Seitenplatten (5) umfasst, die vom Haltekranz (10) über die Schale (1) hinaus überstehen.

5. Schutzvorrichtung nach einem der Ansprüche 1 bis 4, **dadurch gekennzeichnet, dass** die Schale (1) mindestens ein Lüftungsloch (14) umfasst, das in ihren Teil gebohrt ist, der als obenliegend betrachtet wird, sie an einem sich aufrecht befindenden Patienten angebracht ist.

6. Schutzvorrichtung nach einem der Ansprüche 1 bis 5, **dadurch gekennzeichnet, dass** ein Teil (23) des Umfangsrands der Schale (1) lotrecht zur Öffnung (21) des Haltekranzes (10) offen ist und in der Lage ist, den Durchtritt des Beutels (3) zu ermöglichen, sobald die Vorrichtung an demselben positioniert und die Lasche (4) wieder geschlossen ist.

7. Schutzvorrichtung nach einem der Ansprüche 1 bis 6, **dadurch gekennzeichnet, dass** die Schale (1) und der Haltekranz (10) aus zwei fest miteinander zusammenfügbaren Teilen bestehen, wobei einer der Teile des Haltekranzes als entfernbare Lasche (4) dient.

8. Schutzvorrichtung nach Anspruch 7, **dadurch gekennzeichnet, dass** die zwei Teile, sobald sie zusammengefügt sind, den Beutel (3) vollständig umschließen und bedecken.

9. Vorrichtung nach Anspruch 7 oder 8, **dadurch gekennzeichnet, dass** die Ränder der zwei Teile der Schale (1), die eine Fügenahtlinie (17) derselben bilden, von komplementärer Form sind und sich ineinander einpassen.

10. Verfahren zum Anbringen einer Vorrichtung zum Schutz eines Stomabeutels (3), der an einem Trägerteil (2) befestigt wird, das zwei über ihren Innendurchmesser verbundene Kränze (2₁, 2₂) umfasst, die eine zur Außenseite gerichtete Kehle (15₁) bilden, und von denen einer (2₁) dieser Kränze um die Öffnung (8) eines Stomas herum auf die Haut eines Patienten geklebt wird, und der Beutel (3) mit einer starren und hohlen Schale (1) abgedeckt wird, die fest mit einem Haltekranz oder -ring (10) verbunden ist und dessen mittlerer ausgesparter Teil (22) einerseits von kleinerer Abmessung ist als jener der Außenform des Beutels (3), und andererseits in der Lage ist, den Durchtritt der Einlassöffnung (12) des Beutels (3) zu ermöglichen, wenn derselbe mit dem Trägerteil (2) verbunden wurde, **dadurch gekennzeichnet, dass**:
- der Haltekranz (10), der an einem Teil (21) seines Umfangs offen ist, hinter den Beutel (3) und von oben nach unten geschoben wird, wenn sich der Patient in aufrechter Position befindet, wobei sich so mindestens der obere Teil des Beutels in den mittleren ausgesparten Teil (22) der Schale (1) einfügt,
- das Schieben des Haltekranzes (10) gestoppt wird, wenn dieser um die Kehle (15₁) des Trägerteils (2) herum angeordnet ist, wobei er die Verbindung der Einlassöffnung (12) des Beutels (3) an diesem Trägerteil (2) sicherstellt, und die Vorrichtung dort eingehängt wird,
- die Öffnung (21) des offenen Teils des Umfangs des Haltekranzes (10) mit einer entfernbaren Lasche (4) geschlossen wird, sobald dieser Kranz (10) um die Kehle (15₁) herum angebracht ist, wobei der Kranz (10) und seine entfernbare Lasche (4) in so geschlossener Position die Kehle (15₁) dann vollständig umschließen und jede Möglichkeit des Bewegens der Schutzschale durch seitliches oder vertikales Verschieben verhindern,
- ein Gurt (6) an der Schale (1), und der den Körper des Patienten umschließt, festgemacht wird und so durch Festziehen des Gurtes (6) unter Drücken auf diese Schale der Haltekranz (10) unter sandwichartigem Einfassen des auf die Haut geklebten Kranzes (2₁) des Trägerteils (2) gegen die Haut gepresst wird, wodurch jedes Auslaufrisiko verhindert wird.

## Claims

1. Protection device for an ostomy bag (3) that will be fixed to a support part (2) comprising two rings (2₁, 2₂) connected by their inside diameter forming a groove (15₁) facing outwards, one of rings can be glued around the orifice (8) of an ostomy and on the skin of a patient, said device comprising a rigid, hollow shell (1) capable of covering said bag (3), and a support ring (10) fixed to the edge of said shell (1), and the hollowed out central part (22) of which is firstly smaller than the external shape of the bag (3) and secondly, capable of allowing the passage through the inlet orifice (12) of the bag (3) once the bag is connected to the support part (2), the support ring (10) being open on a part (21) of its periphery and thus making it possible, when the device is put into place on the bag (3), to slide said ring (10) backwards on the bag (3) and from the top when the patient is in the standing up position, and in the groove (15₁) of the support part (2) making the connection between the inlet orifice (12) of the bag (3) on this support part (2) and attaching the device to it, **characterised in that**:
- the support ring (10) comprises a removable tongue (4) that closes the opening (21) of the open part of the periphery of the ring (10) once the ring has been put into place around the groove (15₁), thus making it impossible to move the protection device by sliding it laterally or vertically, said ring (10) and its removable cleat (4) in the closed position being capable of fully surrounding the groove (15₁).

2. Protection device according to claim 1 **characterised in that** it comprises at least one belt (6) that can bear on the shell (1) and push the support ring (10) into contact with the skin (13).

3. Device according to claim 2 **characterised in that** it comprises at least two sets of the belt loops (7) that can be used to adjust the position of the belt (6) relative to the shell (1).

4. Device according to any one of claims 1 to 3 **characterised in that** it comprises lateral plates (5) that project from the support ring (10) beyond the shell (1) .

5. Protection device according to any one of claims 1 to 4 **characterised in that** the shell (1) comprises at least one vent hole (14) drilled in its part considered to be at the top when it is put into place on a patient who is standing up.

6. Protection device according to any one of claims 1 to 5 **characterised in that** a part (23) of the peripheral edge of the shell (1) is open vertically in line with the opening (21) of the support ring (10) and can allow the bag (3) to pass through once the device is in position on the bag and the cleat (4) is closed.

7. Protection device according to any one of claims 1 to 6 **characterised in that** the shell (1) and the support ring (10) are in two parts that can be assembled and fixed to each other, one of the parts of the support ring acting as the removeable cleat (4).

8. Protection device according to claim 7 **characterised in that**, once assembled, the two parts surround and entirely cover the bag (3).

9. Protection device according to claim 7 or 8 **characterised in that** the edges of the two parts of the shell (1) forming the assembly junction line (17) of the shell have complementary shapes and fit into each other.

10. Method of putting a protection device for an ostomy bag (3) into place that is fixed on a support part (2) comprising two rings (2₁, 2₂) connected by their inside diameter forming a groove (15₁) facing outwards, one (2₁) of these rings is glued around the orifice (8) of an ostomy on the skin of a patient, and in which said bag (3) is covered by a hollow rigid shell (1) fixed to a support ring (10) and the hollowed out central part (22) of which is firstly smaller than the external shape of the bag (3) and secondly capable of allowing passage through the inlet orifice (12) of the bag (3) once the bag is connected to the support part (2), **characterised in that**:
- the support ring (10) that is open on a part (21) of its periphery is slid behind the bag (3) and from top to bottom when the patient is in the standing up position, at least the upper part of the bag thus fitting in the hollowed out central part (22) of the shell (1),
- sliding of the support ring (10) is stopped when the ring is in position around the groove (15₁) of the support part (2) making the connection of the inlet orifice (12) of the bag (3) on this support part (2) and the device is attached to it,
- the opening (21) of the open part of the periphery of the support ring (10) is held in place by a removable tongue (4) once this ring (10) has been put into place around the groove (15₁), said ring (10) and its removable tongue (4) in the position thus closed then completely surrounding the groove (15₁) making it impossible to move the protection shell by sliding laterally or vertically,
- a belt (6) is applied on said shell (1) completely surrounding the patient's body and this squeezing the belt (6) onto this shell, the support ring (10) is held in contact with the skin forming a "sandwich" of the ring (2₁) of the support part (2) that is bonded to the skin, thus preventing any risk of a leak.
